# FASCICULE DE BREVET EUROPEEN

(11) **EP 1 235 579 B1**
(45) Date de publication et mention de la délivrance du brevet: **16.07.2003**
(21) Numéro de dépôt: 00981453.4
(22) Date de dépôt: 22.11.2000
(51) Int. Cl.: A61K 31/575, A61K 9/20, A61K 47/00, A61P 15/12, A61P 15/18, A61P 19/10, A61K 31/565

(54) **COMPOSITIONS PHARMACEUTIQUES COMPRENANT DE LA TRIMEGESTONE**
TRIMEGESTONE ENTHALTENDE PHARMAZEUTISCHE ZUSAMMENSETZUNGEN
PHARMACEUTICAL COMPOSITIONS COMPRISING TRIMEGESTONE

(30) Priorité: 23.11.1999 FR 9914714
(43) Date de publication de la demande: 04.09.2002
(73) Titulaire: Aventis Pharma S.A., 92160 Antony (FR)
(72) Inventeur: BECOURT, Philippe, F-91300 Massy (FR); GEORGES, Robert, F-93170 Bagnolet (FR); SEGOT CHICQ, Serge, F-94240 l'Hay les Roses (FR)
(74) Mandataire: Rousseau, Pierrick Edouard
(86) Numéro de dépôt international: FR0003240
(87) Numéro de publication internationale: WO01037841

(56) Documents cités:
- US-A- 5 384 419
- US-A- 5 399 685
- US-A- 5 759 577
- US-A- 5 834 452

## Description

La présente invention a pour objet des compositions pharmaceutiques comprenant de la Trimegestone éventuellement associée à un estrogène, leurs procédés de préparation ainsi que le conditionnement primaire les renfermant.

La Trimegestone est un stéroïde en série norpregnane, la 17alpha-méthyl-17bêta-(2-hydroxy-1-oxo-propyl)-estra-4,9-dièn-3-one (21S) connue pour son activité progestomimétique. Il a été décrit pour la première fois dans la demande Européenne EP-A-007823. De nombreuses publications font état des propriétés très intéressantes de ce progestomimétique (traitement hormonal substitutif de la ménopause, contraception). Ce composé peut être utilisé seul ou en association avec un estrogène : D. Ross et al. Maturitas (1977) 28(1) 83-88 27 (Suppl.) 64- Al-Azzawi et al Hum Reprod. (1999) 14(3) 636-641 - WO 9804269-A1- WO 9804268-A1 ; WO 9804265-A1 ; WO 9804246-A1.

Les compositions pharmaceutiques décrites dans EP-A-0007823 sont notamment celles administrables par voie orale, à savoir les comprimés, les comprimés enrobés, les granulés, les cachets et les gélules. Ces compositions pharmaceutiques peuvent être préparées selon les méthodes classiques connues de l'homme du métier dans le domaine de la galénique, par exemple par granulation humide ou par compression directe. D'autres compositions pharmaceutiques renfermant la Trimegestone sont décrites dans les demandes Européennes EP-0722720-A1 ou EP-0803250-A1.

La Trimegestone , sous forme de poudre, est une molécule très peu sensible à la lumière et est stable après 3 mois de conservation à 70°C sous gaz inerte ou après 6 mois de conservation dans un pilulier verre à 50°C. La Trimegestone est donc une molécule stable en tant que telle. Cependant, des problèmes de stabilité peuvent apparaître lors de la préparation des comprimés ou lorsque les compositions pharmaceutiques classiques renfermant Trimegestone sont conservées à 40°C ( milieu sec ou humide) ou dans des conditions plus sévères. On observe notamment dans ces conditions l'apparition du 17alpha-méthyl-7bêta-(1-hydroxy-2-oxo-propyl)-estra-4,9-dièn-3-one (20S), de l'épimère 21R de la Trimégestone ou encore de l'oxopromégestone.

Afin d'éviter tout problème de stabilité, et afin de répondre aux normes en vigueur dans le domaine des compositions pharmaceutiques, la demanderesse a mis au point une nouvelle composition pharmaceutique qui repose essentiellement sur l'ajout d'un tampon dont le pH est compris entre 2 et 5,5 ainsi qu'un procédé de granulation humide original. D'autres additifs pourront être avantageusement ajoutés pour améliorer la stabilité ainsi que d'autres propriétés requises pour un comprimé.

L'invention a donc pour objet une composition pharmaceutique, sous forme solide, destinée à une administration par voie orale, renfermant de la Trimégestone et éventuellement un ou plusieurs estrogènes caractérisée en ce qu'elle comprend un tampon dont le pH est compris essentiellement entre 2 et 5,5. La mesure du pH s'effectue au moment de l'incorporation du tampon lors de la mise en oeuvre du procédé de préparation.

Parmi les tampons permettant d'obtenir un pH compris entre 2 et 5,5, on peut citer notamment le tampon formé par la combinaison d'acide citrique et de phosphate disodique lesdits composés étant le cas échéant sous forme hydratée (monohydrate ou dihydrate). De préférence, le pH du tampon est essentiellement égal à 3.

L'invention a donc plus particulièrement pour objet une composition pharmaceutique telle que définie précédemment caractérisée en ce que le tampon est constitué d'un mélange d'acide citrique et de phosphate disodique.

L'invention a tout particulièrement pour objet une composition pharmaceutique telle que définie précédemment caractérisée en ce que le mélange d'acide citrique et de phosphate disodique présente un pH essentiellement égal à 3.

La Trimegestone peut être associée à un estrogène. Cet estrogène peut être le 17bêta-estradiol (estradiol), l'estradiol valérate, l'éthynylestradiol, le mestranol, le quinestranol, l'oestrone, un estrogène conjugué ou estérifié, l'estropipate ou encore un estrogène d'origine équine tel que le Premarin®, les dits composés pouvant être le cas échéant sous forme hydratée.

L'invention a donc plus particulièrement pour objet la composition pharmaceutique telle que définie précédemment renfermant de la Trimegestone et un estrogène.

L'invention a plus particulièrement pour objet la composition pharmaceutique telle que définie précédemment dans laquelle l'estrogène est le 17bêta-estradiol, notamment sous forme hemihydrate.

L'invention a également plus particulièrement pour objet la composition pharmaceutique telle que définie précédemment caractérisée en ce que l'estrogène est le Prémarin.

Plusieurs additifs peuvent être prévus dans cette composition pharmaceutique. On peut ainsi de manière avantageuse incorporer un ou plusieurs agents liants, un ou plusieurs agents lubrifiants, un ou plusieurs agents opacifiants, un ou plusieurs agents de chélation et un ou plusieurs agents diluants.

Parmi les agents liants, on peut citer les dérivés cellulosiques tels que l'hydroxypropylmethylcellulose basse viscosité (H.P.M.C), l'hydroxypropylcellulose (H.P.C.), la carboxyméthylcellulose (C.M.C.), la méthylcellulose (M.C), ou encore la polyvidone, l'amidon ou la gélatine. A titre préféré, l'agent liant sera un dérivé cellulosique, notamment l'H.P.M.C.

L'invention a donc plus particulièrement pour objet la composition pharmaceutique telle que définie précédemment caractérisée en ce qu'elle comprend en outre un ou plusieurs agents liants, notamment un dérivé cellulosique.

L'invention a donc tout particulièrement pour objet la composition pharmaceutique telle que définie précédemment caractérisée en ce que le dérivé cellulosique est l'HPMC.

Parmi les agents lubrifiants, on peut citer, le stéarate de magnésium, de calcium ou de Zinc, l'acide stéarique, le stéaryl fumarate de sodium, le polyéthylène glycol (P.E.G.), le talc, les esters d'acides gras tels que le COMPRITOL® ou le MYVATEX®. A titre préféré, l'agent lubrifiant sera l'acide stéarique et/ou le talc.

L'invention a donc tout particulièrement pour objet la composition pharmaceutique telle que définie précédemment caractérisée en ce qu'elle comprend en outre un ou plusieurs agents lubrifiants, notamment l'acide stéarique et/ou le talc.

L'invention a également tout particulièrement pour objet la composition pharmaceutique telle que définie précédemment caractérisée en ce qu'elle comprend en outre un ou plusieurs agents opacifiants, notamment le TiO₂.

Parmi les agents de chélation, on peut citer l'acide citrique, l'éthylène diamine et la phénylalanine.
L'invention a donc tout particulièrement pour objet la composition pharmaceutique telle que définie précédemment caractérisée en ce qu'elle comprend en outre un ou plusieurs agents de chélation, notamment l'EDTA.

Parmi les agents diluants, on peut citer, l'amidon de mais, l'amidon de riz, l'amidon de pomme de terre, le lactose, le mannitol, la cellulose et les dérivés calciques tels que le phosphate dicalcique. A titre préféré, l'agent diluant est l'amidon de mais et/ou le lactose.

L'invention a donc tout particulièrement pour objet la composition pharmaceutique telle que définie précédemment caractérisé en ce qu'elle comprend en outre un ou plusieurs agents diluants tel que l'amidon de maïs et/ou le lactose.

L'invention a donc tout particulièrement pour objet une composition pharmaceutique telle que définie précédemment caractérisée en ce qu'elle comprend :
a) la Trimegestone éventuellement associée avec un estrogène, notamment l'estradiol,
b) un tampon constitué d'acide citrique et de phosphate disodique dont le pH, lors de la mise en oeuvre du procédé de préparation, est essentiellement égal à 3,
c) le cas échéant un ou plusieurs agents liants,
d) le cas échéant un ou plusieurs agents lubrifiants,
e) le cas échéant un ou plusieurs agents opacifiants,
f) le cas échéant un ou plusieurs agents de chélation,
g) un ou plusieurs agents diluants.

L'invention a tout particulièrement pour objet une composition pharmaceutique telle que définie précédemment caractérisée en ce qu'elle comprend (% en poids)
. 0,05% à 3% de Trimégestone
. 0,30% à 6% d'estrogène
. 0% à 6% d'un agent liant tel que l'HPMC
. 0% à 2% d'un agent opacifiant tel que le dioxyde de titane
. 0,1% à 2% d'un tampon phosphate disodique/acide citrique dont le pH lors de la mise en oeuvre du procédé de préparation est essentiellement égal à 3
. 0% à 4% d'un agent lubrifiant tel que l'acide stéarique et/ou le talc
. 0% à 0,2% d'un agent de chélation tel que l'EDTA
. qs de diluant.

L'invention a tout particulièrement pour objet une composition pharmaceutique telle que définie précédemment caractérisée en ce qu'elle renferme de 0,2 à 0,8% de Trimégestone.

L'invention a tout particulièrement pour objet une composition pharmaceutique telle que définie précédemment caractérisé en ce qu'elle comprend (% en poids)
- 0,4% de Trimegestone, 3,2% d'estradiol, 4% d'HPMC, 1% de dioxyde de titane, 0,15% phosphate disodium anhydre, 0,4% acide citrique monohydrate, 0,6% d'acide stéarique, 1,2% de talc, 0,1% d'EDTA, 31% d'amidon de maïs, qs lactose.
- 0,8% de Trimegestone, 3,2% d'estradiol, 4% d'HPMC, 1% de dioxyde de titane, 0,15% phosphate disodium anhydre, 0,4% acide citrique monohydrate, 0,6% d'acide stéarique, 1,2% de talc et 0,1% d'EDTA, 31% d'amidon de maïs, qs lactose.
- 0,25 mg de Trimegestone, 2,0 mg d'estradiol, 2,5 mg d'HPMC, 0,6 mg de dioxyde de titane, 0,1 mg phosphate disodium anhydre, 0,25 mg acide citrique monohydrate, 20 mg d'amidon de mais, 38,1 mg de lactose, 0,4 mg d'acide stéarique, 0,8 mg de talc et 0,065 mg d'EDTA
- 0,5 mg de Trimegestone, 2,0 mg d'estradiol, 2,5 mg d'HPMC, 0,6 mg de dioxyde de titane, 0,1 mg phosphate disodium anhydre, 0,25 mg acide citrique monohydrate, 20 mg d'amidon de mais, 37,85 mg de lactose, 0,4 mg d'acide stéarique, 0,8 mg de talc et 0,065 mg d'EDTA.

Un autre aspect de l'invention est le procédé de préparation des composés selon l'invention. On opère en utilisant la technique de granulation humide.

L'invention a plus particulièrement pour objet le procédé de préparation des comprimés selon l'invention comprenant les étapes suivantes :
1) Obtention d'un granulé d'estrogène et de Trimégestone
   a) mélange de l'estrogène et de la trimégestone avec un agent liant et un ou plusieurs diluants puis
   b) mouillage par ajout d'une suspension aqueuse contenant le tampon tel que défini précédemment, un agent liant et un agent opacifiant
   c) séchage
   d) calibrage
2) Lubrification et,
3) Compression.

Selon un mode de réalisation préféré concernant la composition renfermant les deux principes actifs, on prépare préalablement deux granulés renfermant respectivement la Trimegestone et un estrogène, les deux granulés étant mélangés préalablement à l'étape de lubrification.

L'invention a donc également pour objet le procédé de préparation des comprimés selon l'invention comprenant les étapes suivantes :
1) Obtention d'un granulé d'estrogène
   a) mélange de l'estrogène avec un agent liant et un ou plusieurs diluants puis
   b) mouillage par ajout d'une suspension aqueuse contenant le tampon tel que défini précédemment, un agent liant et un agent opacifiant
   c) séchage
   d) calibrage
2) Obtention d'un granulé de Trimégestone
   a) mélange d'un agent liant et un ou plusieurs diluant puis
   b) mouillage par ajout
      - d'une suspension aqueuse contenant le tampon, un agent liant et un agent opacifiant,
      - de la Trimegestone
   c) séchage
   d) calibrage
3) Mélange des granulés d'estrogène et de Trimegestone
4) Lubrification et,
5) Compression.

Le conditionnement primaire peut être le suivant :

Les comprimés objets de l'invention peuvent être insérés dans des plaquettes thermoformées (encore appelées blisters). Un autre aspect de l'invention concerne donc ces plaquettes thermoformées renfermant des comprimés de Trimegestone éventuellement associée à un estrogène, selon l'invention. Ces comprimés sont notamment au nombre de 28.

A titre préféré, les plaquettes thermoformées sont également insérées dans une sache sous atmosphère inerte, et notamment sous atmosphère d'azote.

Ces compositions pharmaceutiques présentent un intérêt dans le traitement des symptômes de la ménopause et la prévention de l'ostéoporose post-ménopause. Elle peuvent également être utilisées à titre de contraceptif.

L'invention a donc pour objet l'application de la Trimegestone éventuellement associée avec un estrogène, pour la préparation d'une composition pharmaceutique selon l'invention, destinée au traitement des symptômes de la ménopause et la prévention de l'ostéoporose.

Différentes méthodes d'administration sont décrites dans l'exemple 6, et l'invention a pour objet l'application de la Trimegestone éventuellement associée avec un estrogène, pour la préparation d'une composition pharmaceutique selon l'invention, destinée au traitement des symptômes de la ménopause dans lequel le dit traitement s'effectue par administration orale selon l'une quelconque des méthodes a) à e) :
a)
   - d'un comprimé estradiol en continu à une dose de 0,5 à 2 mg par jour les 14 à 18 premiers jours,
   - et du comprimé selon l'invention renfermant la Trimégestone (0.025 à 2 mg par jour) et l'estradiol (0,5 à 2 mg par jour) les 10 à 14 derniers jours de chaque cycle de 28 jours. (cycles de 28 jours sans interruption entre les cycles),
b)
   - d'un comprimé estradiol à une dose de 0,5 à 2 mg par jour les 14 à 18 premiers jours,
   - et du comprimé selon l'invention renfermant la Trimégestone (0,025 à 2 mg par jour) et l'estradiol (0,5 à 2 mg par jour) les 10 à 14 derniers jours, le traitement étant arrêté 2 à 3 jours par mois à la fin de chaque cycle de 28 jours. (cycle de 28 jours par mois),
c)
   - du comprimé selon l'invention renfermant la Trimégestone (0,025 à 2 mg par jour) et l'estradiol (0,5 à 2 mg par jour) les 10 à 14 premiers jours,
   - et d'un comprimé estradiol, à une dose de 0,5 à 2 mg par jour les 14 à 18 derniers jours, le traitement étant administré soit sans interruption entre chaque cycle de 28 jours, soit avec interruption de 2 à 3 jours par mois à la fin ce chaque cycle,
d)
   - d'un comprimé estradiol les 14 premiers jours, à une dose de 0,5 à 2 mg par jour,
   - et du comprimé selon l'invention renfermant la Trimégestone (0,025 à 2 mg par jour) et l'estradiol (0,5 à 2 mg par jour) pendant les 11 derniers jours, Le traitement étant arrêté 5 à 6 jours à la fin de chaque cycle de 25 jours,
e)
   - du comprimé selon l'invention renfermant la Trimégestone (0,025 à 2 mg par jour) et l'estradiol (0,5 à 2 mg par jour) par jour sans interruption de traitement.

L'invention a également pour objet l'application de la Trimegestone associée avec un estrogène, pour la préparation d'une composition pharmaceutique selon l'invention, comme contraceptif. Dans ce cas on utilisera une méthode d'administration de la composition estro-progestative pendant 21 à 25 jours et 7 à 3 jours d'interruption.

L'invention a également pour objet une méthode de contraception par administration par voie orale des compositions pharmaceutiques selon l'invention.

Les exemples ci-dessous illustrent l'invention sans toutefois la limiter.

### Exemple 1 : Comprimé de trimegestone dosé à 0,25mg

- 0,25 mg de Trimegestone, 2,0 mg d'estradiol, 2,5 mg d'HPMC, 0,6 mg de dioxyde de titane, 0,1 mg phosphate disodium anhydre, 0,25 mg acide citrique monohydrate, 20 mg d'amidon de maïs, 38,1 mg de lactose, 0,4 mg d'acide stéarique, 0,8 mg de talc et 0,065 mg d'EDTA.

### Exemple 2 : Comprimé de trimegestone dosé à 0,5mg

- 0,5 mg de Trimegestone, 2,0 mg d'estradiol, 2,5 mg d'HPMC, 0,6 mg de dioxyde de titane, 0,1 mg phosphate disodium anhydre, 0,25 mg acide citrique monohydrate, 20 mg d'amidon de maïs, 37,85 mg de lactose, 0,4 mg d'acide stéarique, 0,8 mg de talc et 0,065 mg d'EDTA.

### Exemple 3 : Préparation de comprimés renfermant 0,500 mg de Trimégestone et 2,0 mg d'estradiol.

### A) Granulé de Trimegestone (0,500mg)

### . Etape 1 : Préparation de la solution tampon

On ajoute à 10 litres d'eau, 0,080kg de Na₂HPO₄ et 0,200 kg d'acide citrique monohydrate et agite jusqu'à dissolution complète.

### . Etape 2 : Préparation de la solution B

On ajoute à la solution précédente (6,170 kg) 0,640 kg de méthylhydroxypropylcellulose 3cp et agite jusqu'à dissolution complète.

### . Etape 3 : Préparation de la suspension C

On ajoute à la solution B, 0,800 kg de Trimegestone micronisée et 0,480 kg de dioxyde de titane micronisé et maintient l'agitation jusqu'à dispersion complète.

### . Etape 4 : Mélange sec de poudres

On mélange les constituants suivants :
Méthylhydroxypropylcellulose (H.P.M.C.) 3cp (1,360 kg), amidon de maïs (16,400 kg) et du lactose monohydrate (51,040 kg).

### . Etape 5 Granulation humide

On verse la suspension de l'étape 3 (8,090 kg) sur le mélange de poudre de l'étape 4 et rajoute 4,11 kg de la solution restante de l'étape 1.

### · Etape 6

Le granulé humide est ensuite séché à 50°C puis calibré.

### B) Granulé d'Estradiol (2mg)

On opère comme pour la Trimegestone , cependant, l'introduction d'Estradiol s'effectue lors de l'étape 4. 3,2 kg d'Estradiol sont alors utilisés.

### C) Préparation des comprimés renfermant les deux principes actifs (Etape de Lubrification)

On mélange les granulés humides de 0,500 mg de Trimegestone (51,04 kg) et de 2 mg d'Estradiol (51,04kg) puis introduit le mélange constitué de 0,640 kg d'acide stéarique micronisé et de 1,28 kg de Talc.

### Exemple 4

### Essai comparatif de stabilité

Comprimé a (tampon pH 3): Trimegestone 1mg, Estradiol hemohydrate 2,0747 mg, Hypromellose 3 cps 3,0 mg, Oxyde de Titanium 0,6 mg, phosphate disodique 0,0874 mg, acide citrique monohydrate 0,2504 mg, amidon de mais 22 mg lactose 44,4875 mg, stéarate de magnésium 0,5 mg et talc 1,0 mg : masse totale 75,0 mg.

Comprimé b (tampon pH 5) : Trimegestone 1 mg, Estradiol hemohydrate 2,0747 mg, Hypromellose 3 cps 3,0 mg, Oxyde de Titanium 0,6 mg, phosphate disodique 0,219 mg, acide citrique monohydrate 0,153 mg, amidon de maïs 22 mg, lactose 44,4533 g, stéarate de magnésium 0,5 mg et talc 1,0 mg : masse totale 75,0 mg.

Ces deux comprimés sont maintenus 15 jours à 70°C dans une fiole fermée par un bouchon en polyéthylène. Le but de cette étude est de déterminer par HPLC, les produits de dégradations , notamment le (17bêta-(1-hydroxy-2-oxo-propyl)-estra-4,9-dièn-3-one (20S) (produit X). Le seuil de détection est de 0,3%).

| | Comprimé a (Tampon pH 3) | Comprimé b (Tampon pH 5) |
|---|---|---|
| Produit X | Non Détecté | 0,85% |
| Somme des impuretés | 1,70 | 3,25 |

Cette étude tend a démontrer que la stabilité n'est pas due uniquement à la présence d'acide citrique mais également à un effet pH.

### Exemple 5 : Composition du blister et de la sache

Les plaquettes thermoformées sont notamment constituées
- d'une feuille de polyvinyl(chlorure-acétate) transparente (PVC)de 200µm d'épaisseur, et
- d'une feuille d'aluminium constituée de la face extérieure à la face intérieure :
   - d'une laque de protection (nitrocellulose)
   - d'une feuille d'aluminium de 20µm d'épaisseur,
   - d'un verni bleu thermo-scellable (vinyle/acryle)
   - d'un verni incolore thermo-scellable (vinyle/acryle).

Par ailleurs, la sache est notamment une sache tri-couche constituée de la face extérieure à la face intérieure :
- d'un film en polyuréthane
- d'une feuille d'aluminium de 9µm d'épaisseur
- d'un agent laminant (polyuréthane)
- d'un film en polyuréthane de 40µm d'épaisseur.

### Exemple 6 : Schéma d'administration de l'association Trimégestone / estradiol dans le cadre du traitement des symptômes post-ménopausique et de la prévention de l'ostéoporose

Administration orale d'un comprimé estradiol en continu (cycles de 28 jours sans interruption entre les cycles) à une dose de 0,5 à 2 mg par jour et du comprimé selon l'invention renfermant la Trimégestone les 10 à 14 derniers jours de chaque cycle de 28 jours, à une dose de 0,05 à 2 mg par jour.

Administration orale d'un comprimé estradiol 28 jours par mois à une dose de 0,5 à 2 mg par jour et du comprimé selon l'invention renfermant la Trimégestone les 10 à 14 derniers jours de l'administration de l'estradiol, à une dose de 0,05 à 2 mg par jour. Le traitement est arrêté 2 à 3 jours par mois à la fin de chaque cycle de 28 jours.

Administration orale d'un comprimé estradiol 28 jours par mois, à une dose de 0,5 à 2 mg par jour et du comprimé selon l'invention renfermant la Trimégestone les 14 à 18 premiers jours de l'administration de l'estradiol, à une dose de 0,05 à 2 mg par jour. Le traitement est administré soit sans interruption entre chaque cycle de 28 jours, soit avec interruption de 2 à 3 jours par mois à la fin ce chaque cycle.

Administration orale de l'estradiol 25 jours par mois à une dose de 0,5 à 2 mg par jour et de la Trimégestone à une dose de 0,05 à 2,5 mg par jour pendant les 11 derniers jours de l'administration de l'estradiol. Le traitement est arrêté 5 à 6 jours à la fin de chaque cycle de 25 jours.

Administration continue orale de l'estradiol à une dose de 0,5 à 2mg par jour et de la Trimégestone à une dose de 0,05 à 2,5 mg par jour. Il n'y a pas d'interruption de traitement.

Administration continue orale de l'estradiol à une dose de 0,5 à 2 mg par jour et de la Trimégestone à une dose de 0,025 g pendant 21 à 25 jours puis arrêt 7 à 3 jours.

Dans toutes ces méthodes de traitement, lorsqu'on doit administrer simultanément les deux principes actifs, on peut utiliser :
- soit un comprimé Estradiol classique et un comprimé Trimegestone selon l'invention,
- soit un comprimé mixte estradiol/trimégestone selon l'invention.

## Revendications

1. Composition pharmaceutique, sous forme solide, destinée à une administration par voie orale, renfermant de la Trimégestone et éventuellement un ou plusieurs estrogènes **caractérisée en ce qu'**elle comprend un tampon dont le pH est compris essentiellement entre 2 et 5,5.

2. Composition pharmaceutique telle que définie à la revendication 1 **caractérisée en ce que** le tampon est constitué d'un mélange d'acide citrique et de phosphate disodique.

3. Composition pharmaceutique telle que définie à la revendication 2 **caractérisée en ce que** le mélange d'acide citrique et de phosphate disodique présente un pH essentiellement égal à 3.

4. Composition pharmaceutique telle que définie à l'une quelconque des revendications 1 à 3 **caractérisée en ce qu'**elle renferme de la Trimégestone et un estrogène.

5. Composition pharmaceutique telle que définie à la revendication 4 **caractérisée en ce que** l'estrogène est le 17-bêta-estradiol, notamment sous forme hemihydrate.

6. Composition pharmaceutique telle que définie à la revendication 4 **caractérisée en ce que** l'estrogène est le Prémarin.

7. Composition pharmaceutique telle que définie à l'une quelconque des revendications 1 à 6 **caractérisée en ce qu'**elle comprend en outre un ou plusieurs agents liants.

8. Composition pharmaceutique telle que définie à la revendication 7 **caractérisée en ce que** l'agent liant est un dérivé cellulosique.

9. Composition pharmaceutique telle que définie à la revendication 8 **caractérisée en ce que** le dérivé cellulosique est l'HPMC.

10. Composition pharmaceutique telle que définie à l'une quelconque des revendications 1 à 9 **caractérisée en ce qu'**elle comprend en outre un ou plusieurs agents lubrifiants.

11. Composition pharmaceutique telle que définie à la revendication 10 **caractérisée en ce que** l'agent lubrifiant est l'acide stéarique et/ou le talc.

12. Composition pharmaceutique telle que définie à l'une quelconque des revendications 1 à 11 **caractérisée en ce qu'**elle comprend en outre un ou plusieurs agents opacifiants, notamment le TiO₂.

13. Composition pharmaceutique telle que définie à l'une quelconque des revendications 1 à 12 **caractérisée en ce qu'**elle comprend en outre un ou plusieurs agents de chélation.

14. Composition pharmaceutique telle que définie à la revendication 13 **caractérisée en ce que** l'agent de chélation est l'EDTA.

15. Composition pharmaceutique telle que définie à l'une quelconque des revendications 1 à 14 **caractérisée en ce qu'**elle comprend en outre un ou plusieurs agents diluants tel que l'amidon de mais et/ou le lactose.

16. Composition pharmaceutique telle que définie à l'une quelconque des revendications 1 à 15 **caractérisée en ce qu'**elle comprend :
a) la Trimegestone éventuellement associée avec un estrogène, notamment l'estradiol,
b) un tampon constitué d'acide citrique et de phosphate disodique dont le pH est essentiellement égal à 3
c) le cas échéant un ou plusieurs agents liants,
d) le cas échéant un ou plusieurs agents lubrifiants,
e) le cas échéant un ou plusieurs agents opacifiants,
f) le cas échéant un ou plusieurs agents de chélation,
g) un ou plusieurs agents diluants.

17. Composition pharmaceutique telle que définie à l'une quelconque des revendications 1 à 16 **caractérisée en ce qu'**elle comprend (% en poids)
. 0,05% à 3% de Trimegestone
. 0,30% à 6% d'estrogène
. 0% à 6% d'un agent liant tel que l'HPMC
. 0% à 2% de dioxyde de titane
. 0,1% à 2% d'un tampon constitué d'acide citrique et de phosphate disodique dont le pH est essentiellement égal à 3
. 0% à 4% d'acide stéarique et/ou de talc
. 0% à 0,2% d'un agent de chélation tel que l'EDTA
. qs de diluant

18. Composition pharmaceutique telle que définie à la revendication 17 **caractérisée en ce qu'**elle renferme de 0,2 à 0,8% de Trimégestone.

19. Composition pharmaceutique telle que définie à l'une quelconque des revendications 1 à 18 **caractérisée en ce qu'**elle comprend (% en poids)
. 0,4% de Trimegestone
. 3,2% d'estradiol
. 4% d'HPMC
. 1% de dioxyde de titane
. 0,15% phosphate disodium anhydre
. 0,4% acide citrique monohydrate
. 31% d'amidon de mais
. 0,6% d'acide stéarique
. 1,2% de talc
. 0,1% d'EDTA
. qs lactose

20. Composition pharmaceutique telle que définie à à l'une quelconque des revendications 1 à 18 **caractérisée en ce qu'**elle comprend (% en poids)
. 0,8% de Trimegestone
. 3,2% d'estradiol
. 4% d'HPMC
. 1% de dioxyde de titane
. 0,15% phosphate disodium anhydre
. 0,4% acide citrique monohydrate
. 31% d'amidon de maïs
. 0,6% d'acide stéarique
. 1,2% de talc
. 0,1% d'EDTA
. qs lactose.

21. Composition pharmaceutique de 65 mg telle que définie à l'une quelconque des revendications 1 à 18 **caractérisé en ce qu'**elle comprend
. 0,25 mg de Trimegestone
. 2,0 mg d'estradiol
. 2,5 mg d'HPMC
. 0,6 mg de dioxyde de titane
. 0,1 mg phosphate disodium anhydre
. 0,25 mg acide citrique monohydrate
. 20 mg d'amidon de maïs
. 38,1 mg de lactose
. 0,4 mg d'acide stéarique
. 0,8 mg de talc
. 0,065 mg d'EDTA.

22. Composition pharmaceutique de 65 mg telle que définie à l'une quelconque des revendications 1 à 18 **caractérisée en ce qu'**elle comprend
. 0,5 mg de Trimegestone
. 2,0 mg d'estradiol
. 2,5 mg d'HPMC
. 0,6 mg de dioxyde de titane
. 0,1 mg phosphate disodium anhydre
. 0,25 mg acide citrique monohydrate
. 20 mg d'amidon de maïs
. 37,85 mg de lactose
. 0,4 mg d'acide stéarique
. 0,8 mg de talc
. 0,065 mg d'EDTA.

23. Procédé de préparation des compositions pharmaceutiques telles que définies à l'un quelconque des revendications 1 à 22 **caractérisé en ce qu'**on opère par granulation humide.

24. Procédé selon la revendication 23 comprenant les étapes suivantes :
1) Obtention d'un granulé d'estrogène et de Trimégestone
a) mélange de l'estrogène et de la trimégestone avec un agent liant et un ou plusieurs diluants puis
b) mouillage par ajout d'une suspension aqueuse contenant le tampon tel que défini à l'une quelconque des revendications 1 à 3, un agent liant et un agent opacifiant
c) séchage
d) calibrage
2) Lubrification, et
3) Compression.

25. Procédé selon la revendication 24 **caractérisé en ce que** l'on prépare préalablement deux granulés renfermant respectivement la Trimégestone et un estrogène, les deux granulés étant mélangés préalablement à l'étape de "lubrification".

26. Procédé selon la revendication 25 comprenant les étapes suivantes :
1) Obtention d'un granulé d'estrogène
a) mélange de l'estrogène avec un agent liant et un ou plusieurs diluants puis
b) mouillage par ajout d'une suspension aqueuse contenant le tampon tel que défini à l'une quelconque des revendications 1 à 3, un agent liant et un agent opacifiant
c) séchage
d) calibrage
2) Obtention d'un granulé de Trimégestone
a) mélange d'un agent liant et un ou plusieurs diluant puis
b) mouillage par ajout
- d'une suspension aqueuse contenant le tampon, un agent liant et un agent opacifiant,
- de la Trimegestone
c) séchage
d) calibrage
3) Mélange des granulés d'estrogène et de Trimegestone
4) Lubrification, et
5) Compression.

27. Plaquette thermoformée comprenant les comprimés tels que définis à l'une quelconque des revendications 1 à 22.

28. Plaquette thermoformée telle que définie à la revendication 27 **caractérisée en ce qu'**elle est insérée dans une sache sous atmosphère inerte, et notamment sous azote.

29. Application de la Trimegestone éventuellement associée avec un estrogène, pour la préparation d'une composition pharmaceutique telle que définie à l'une quelconque des revendications 1 à 22, destinée au traitement des symptômes de la ménopause et la prévention de l'ostéoporose.

30. Application selon la revendication 29 de la Trimegestone éventuellement associée avec un estrogène, pour la préparation d'une composition pharmaceutique telle que définie à l'une quelconque des revendications 1 à 22, destinée au traitement des symptômes de la ménopause dans laquelle le dit traitement s'effectue par administration orale, selon l'une quelconque des méthodes a) à e) :
a)
- d'un comprimé estradiol en continu à une dose de 0,5 à 2 mg par jour les 14 à 18 premiers jours,
- et du comprimé tel que défini à la revnedication 1 renfermant la Trimégestone (0,025 à 2 mg par jour) et l'estradiol (0,5 à 2 mg par jour) les 10 à 14 derniers jours de chaque cycle de 28 jours. (cycles de 28 jours sans interruption entre les cycles)
b)
- d'un comprimé estradiol à une dose de 0,5 à 2 mg par jour les 14 à 18 premiers jours
- et du comprimé tel que défini à la revendication 1 renfermant la Trimégestone (0,025 à 2 mg par jour) et l'estradiol (0,5 à 2 mg par jour) les 10 à 14 derniers jours, le traitement étant arrêté 2 / 3 jours par mois à la fin de chaque cycle de 28 jours. (cycle de 28 jours par mois)
c)
- du comprimé tel que défini à la revendication 1 renfermant la Trimégestone (0,025 à 2 mg par jour) et l'estradiol (0,5 à 2 mg par jour) les 10 à 14 premiers jours,
- et d'un comprimé estradiol, à une dose de 0,5 à 2 mg par jour les 14 à 18 derniers jours, le traitement étant administré soit sans interruption entre chaque cycle de 28 jours, soit avec interruption de 2 à 3 jours par mois à la fin ce chaque cycle.
d)
- d'un comprimé estradiol les 14 premiers jours,à une dose de 0,5 à 2 mg par jour
- et de du comprimé tel que défini à la revendication 1 renfermant la Trimégestone (0,025 à 2 mg par jour) et l'estradiol (0,5 à 2 mg par jour) pendant les 11 derniers jours, le traitement étant arrêté 5 à 6 jours à la fin de chaque cycle de 25 jours
e)
- du comprimé tel que défini à la revendication 1 renfermant la Trimégestone (0,025 à 2 mg par jour) et l'estradiol (0,5 à 2 mg par jour) par jour sans interruption de traitement.

31. Application de la Trimegestone associée avec un estrogène, pour la préparation d'une composition pharmaceutique telle que définie à l'une quelconque des revendications 1 à 22, comme contraceptif.

## Patentansprüche

1. Pharmazeutische Zusammensetzung in fester Form, die für eine orale Verabreichung bestimmt ist und die Trimegeston und gegebenenfalls ein Estrogen oder mehrere Estrogene enthält, **dadurch gekennzeichnet, daß** sie einen Puffer, dessen pH im wesentlichen zwischen 2 und 5,5 liegt, umfaßt.

2. Pharmazeutische Zusammensetzung nach Anspruch 1, **dadurch gekennzeichnet, daß** der Puffer aus einem Gemisch aus Zitronensäure und Dinatriumhydrogenphosphat besteht.

3. Pharmazeutische Zusammensetzung nach Anspruch 2, **dadurch gekennzeichnet, daß** das Gemisch aus Zitronensäure und Dinatriumhydrogenphosphat im wesentlichen einen pH von 3 aufweist.

4. Pharmazeutische Zusammensetzung nach einem der Ansprüche 1 bis 3, **dadurch gekennzeichnet, daß** sie Trimegeston und ein Estrogen enthält.

5. Pharmazeutische Zusammensetzung nach Anspruch 4, **dadurch gekennzeichnet, daß** das Estrogen 17-beta-Estradiol, insbesondere in Form des Hemihydrats, ist.

6. Pharmazeutische Zusammensetzung nach Anspruch 4, **dadurch gekennzeichnet, daß** das Estrogen Premarin ist.

7. Pharmazeutische Zusammensetzung nach einem der Ansprüche 1 bis 6, **dadurch gekennzeichnet, daß** sie außerdem ein oder mehrere Bindemittel umfaßt.

8. Pharmazeutische Zusammensetzung nach Anspruch 7, **dadurch gekennzeichnet, daß** das Bindemittel ein Cellulose-Derivat ist.

9. Pharmazeutische Zusammensetzung nach Anspruch 8, **dadurch gekennzeichnet, daß** das Cellulose-Derivat HPMC ist.

10. Pharmazeutische Zusammensetzung nach einem der Ansprüche 1 bis 9, **dadurch gekennzeichnet, daß** sie außerdem ein oder mehrere Gleitmittel umfaßt.

11. Pharmazeutische Zusammensetzung nach Anspruch 10, **dadurch gekennzeichnet, daß** das Gleitmittel Stearinsäure und/oder Talk ist.

12. Pharmazeutische Zusammensetzung nach einem der Ansprüche 1 bis 11, **dadurch gekennzeichnet, daß** sie außerdem ein oder mehrere Trübungsmittel, insbesondere TiO₂, umfaßt.

13. Pharmazeutische Zusammensetzung nach einem der Ansprüche 1 bis 12, **dadurch gekennzeichnet, daß** sie außerdem einen oder mehrere Chelatbildner umfaßt.

14. Pharmazeutische Zusammensetzung nach Anspruch 13, **dadurch gekennzeichnet, daß** der Chelatbildner EDTA ist.

15. Pharmazeutische Zusammensetzung nach einem der Ansprüche 1 bis 14, **dadurch gekennzeichnet, daß** sie außerdem ein oder mehrere Verdünnungsmittel wie z.B. Maisstarke und/oder Lactose umfaßt.

16. Pharmazeutische Zusammensetzung nach einem der Ansprüche 1 bis 15, **dadurch gekennzeichnet, daß** sie umfaßt:
a) Trimegeston, gegebenenfalls in Verbindung mit einem Estrogen, insbesondere Estradiol;
b) einen Puffer, der aus Zitronensäure und Dinatriumhydrogenphosphat besteht und dessen pH im wesentlichen 3 ist;
c) gegebenenfalls ein oder mehrere Bindemittel;
d) gegebenenfalls ein oder mehrere Gleitmittel;
e) gegebenenfalls ein oder mehrere Trübungsmittel;
f) gegebenenfalls einen oder mehrere Chelatbildner;
g) ein oder mehrere Verdünnungsmittel.

17. Pharmazeutische Zusammensetzung nach einem der Ansprüche 1 bis 16, **dadurch gekennzeichnet, daß** sie umfaßt (in Gew.-%):
- 0,05 % bis 3 % Trimegeston,
- 0,30 % bis 6 % Estrogen,
- 0 % bis 6 % eines Bindemittels wie HPMC,
- 0 % bis 2 % Titandioxid,
- 0,1 % bis 2 % eines Puffers, der aus Zitronensäure und Dinatriumhydrogenphosphat besteht und dessen pH im wesentlichen 3 ist,
- 0 % bis 4 % Stearinsäure und/oder Talk,
- 0 % bis 0,2 % eines Chelatbildners wie z.B. EDTA,
- q.s. Verdünnungsmittel.

18. Pharmazeutische Zusammensetzung nach Anspruch 17, **dadurch gekennzeichnet, daß** sie 0,2 bis 0,8 % Trimegeston enthält.

19. Pharmazeutische Zusammensetzung nach einem der Ansprüche 1 bis 18, **dadurch gekennzeichnet, daß** sie umfaßt (in Gew.-%):
- 0,4 % Trimegeston,
- 3,2 % Estradiol,
- 4 % HPMC,
- 1 % Titandioxid,
- 0,15 % wasserfreies Dinatriumhydrogenphosphat,
- 0,4 % Zitronensäuremonohydrat,
- 31 % Maisstärke,
- 0,6 % Stearinsäure,
- 1,2 % Talk,
- 0,1 % EDTA,
- q.s. Lactose.

20. Pharmazeutische Zusammensetzung nach einem der Ansprüche 1 bis 18, **dadurch gekennzeichnet, daß** sie umfaßt (in Gew.-%):
- 0,8 % Trimegeston,
- 3,2 % Estradiol,
- 4 % HPMC,
- 1 % Titandioxid,
- 0,15 % wasserfreies Dinatriumhydrogenphosphat,
- 0,4 % Zitronensäuremonohydrat,
- 31 % Maisstärke,
- 0,6 % Stearinsäure,
- 1,2 % Talk,
- 0,1 % EDTA,
- q.s. Lactose.

21. Pharmazeutische Zusammensetzung mit 65 mg nach einem der Ansprüche 1 bis 18, **dadurch gekennzeichnet, daß** sie umfaßt:
- 0,25 mg Trimegeston,
- 2,0 mg Estradiol,
- 2,5 mg HPMC,
- 0,6 mg Titandioxid,
- 0,1 mg wasserfreies Dinatriumhydrogenphosphat,
- 0,25 mg Zitronensäuremonohydrat,
- 20 mg Maisstärke,
- 38,1 mg Lactose
- 0,4 mg Stearinsäure,
- 0,8 mg Talk,
- 0,065 mg EDTA.

22. Pharmazeutische Zusammensetzung mit 65 mg nach einem der Ansprüche 1 bis 18, **dadurch gekennzeichnet, daß** sie umfaßt:
- 0,5 mg Trimegeston,
- 2,0 mg Estradiol,
- 2,5 mg HPMC,
- 0,6 mg Titandioxid,
- 0,1 mg wasserfreies Dinatriumhydrogenphosphat,
- 0,25 mg Zitronensäuremonohydrat,
- 20 mg Maisstärke,
- 37,85 mg Lactose
- 0,4 mg Stearinsäure,
- 0,8 mg Talk,
- 0,065 mg EDTA.

23. Verfahren zur Herstellung pharmazeutischer Zusammensetzungen, wie sie in einem der Ansprüche 1 bis 22 definiert sind, **dadurch gekennzeichnet, daß** man mit Feuchtgranulierung arbeitet.

24. Verfahren nach Anspruch 23, das die folgenden Stufen umfaßt:
1) Herstellung eines Granulats aus Estrogen und Trimegeston,
a) Vermischen von Estrogen und Trimegeston mit einem Bindemittel und einem oder mehreren Verdünnungsmitteln, dann
b) Anfeuchten durch Zusatz einer wäßrigen Suspension, die den Puffer, wie er in einem der Ansprüche 1 bis 3 definiert ist, ein Bindemittel und ein Trübungsmittel enthält,
c) Trocknung,
d) Kalibrieren,
2) Schmierung und
3) Verpressen.

25. Verfahren nach Anspruch 24, **dadurch gekennzeichnet, daß** man im voraus 2 Granulate, die Trimegeston bzw. ein Estrogen enthalten, herstellt und die zwei Granulate vor der Stufe der "Schmierung" vermischt werden.

26. Verfahren nach Anspruch 25, das die folgenden Stufen umfaßt:
1) Herstellung eines Estrogen-Granulats
a) Vermischen von Estrogen mit einem Bindemittel und einem oder mehreren Verdünnungsmitteln, dann
b) Anfeuchten durch Zusatz einer wäßrigen Suspension, die den Puffer, wie er in einem der Ansprüche 1 bis 3 definiert ist, ein Bindemittel und ein Trübungsmittel enthält,
c) Trocknung,
d) Kalibrieren,
2) Herstellung eines Trimegeston-Granulats
a) Vermischen eines Bindemittels und eines Verdünnungsmittels oder mehrerer Verdünnungsmittel, dann
b) Anfeuchten durch Zusatz
- einer wäßrigen Suspension, die den Puffer, ein Bindemittel und ein Trübungsmittel enthält,
- von Trimegeston,
c) Trocknung,
d) Kalibrierung,
3) Vermischen der Granulate von Estrogen und Trimegaston,
4) Schmierung und
5) Verpressen.

27. Thermogeformtes Plättchen, das die Tabletten, wie sie in einem der Ansprüche 1 bis 22 definiert sind, umfaßt.

28. Thermogeformtes Plättchen nach Anspruch 27, **dadurch gekennzeichnet, daß** es in inerter Atmosphäre und insbesondere unter Stickstoff, in einem Beutel eingeschlossen ist.

29. Verwendung von Trimegeston, gegebenenfalls in Verbindung mit Estrogen, zur Herstellung einer pharmazeutischen Zusammensetzung nach einem der Ansprüche 1 bis 22, die zur Behandlung der Symptome der Menopause und zur Prävention von Osteoporose bestimmt ist.

30. Anwendung nach Anspruch 29 von Trimegeston, gegebenenfalls in Verbindung mit einem Estrogen, zur Herstellung einer pharmazeutischen Zusammensetzung, wie sie in einem der Ansprüche 1 bis 22 definiert ist, die zur Behandlung der Symptome der Menopause bestimmt ist, wobei die Behandlung durch orale Verabreichung nach einem der Verfahren a) bis e) erfolgt:
a)
- eine Estradiol-Tablette fortlaufend mit einer Dosis von 0,5 bis 2 mg pro Tag während der ersten 14 bis 18 Tage,
- die Tablette, wie sie in Anspruch 1 definiert ist, die Trimegeston (0,025 bis 2 mg pro Tag) und Estradiol (0,5 bis 2 mg pro Tag) enthält, die 10 bis 14 letzten Tage jedes Zyklus mit 28 Tagen (Zyklen mit 28 Tagen ohne Unterbrechung zwischen den Zyklen);
b)
- eine Estradiol-Tablette mit einer Dosis von 0,5 bis 2 mg pro Tag während der ersten 14 bis 18 Tage,
- die Tablette, wie sie in Anspruch 1 definiert ist, die Trimegeston (0,025 bis 2 mg pro Tag) und Estradiol (0,5 bis 2 mg pro Tag) enthält, die letzten 10 bis 14 Tage, wobei die Behandlung 2/3 Tage pro Monat am Ende jedes Zyklus mit 28 Tagen angehalten wird (Zyklus mit 28 Tagen pro Monat);
c)
- die Tablette, wie sie in Anspruch 1 definiert ist, die Trimegeston (0,025 bis 2 mg pro Tag) und Estradiol (0,5 bis 2 mg pro Tag) enthält, die ersten 10 bis 14 Tage,
- und eine Estradiol-Tablette mit einer Dosis von 0,5 bis 2 mg pro Tag die letzten 14 bis 18 Tage, wobei die Behandlung entweder ohne Unterbrechung zwischen jedem Zyklus aus 28 Tagen oder mit einer Unterbrechung von 2 bis 3 Tagen pro Monat am Ende jedes Zyklus durchgeführt wird;
d)
- eine Estradiol-Tablette während der ersten 14 Tage mit einer Dosis von 0,5 bis 2 mg pro Tag,
- und die Tablette, wie sie in Anspruch 1 definiert ist, die Trimegeston (0,025 bis 2 mg pro Tag) und Estradiol (0,5 bis 2 mg pro Tag) enthält, während der letzten 11 Tage, wobei die Behandlung 5 bis 6 Tage am Ende jedes Zyklus mit 25 Tagen angehalten wird;
e)
- die Tablette, wie sie in Anspruch 1 definiert ist, die Trimegeston (0,025 bis 2 mg pro Tag) und Estradiol (0,5 bis 2 mg pro Tag) enthält, pro Tag ohne Unterbrechung der Behandlung.

31. Verwendung des Trimegestons in Verbindung mit einem Estrogen zur Herstellung einer pharmazeutischen Zusammensetzung nach einem der Ansprüche 1 bis 22 als Kontrazeptivum.

## Claims

1. Pharmaceutical composition in solid form, intended for oral administration, containing Trimegestone and, optionally, one or more oestrogens, **characterized in that** it comprises a buffer, the pH of which is essentially between 2 and 5.5.

2. Pharmaceutical composition as defined in Claim 1, **characterized in that** the buffer consists of a mixture of citric acid and disodium phosphate.

3. Pharmaceutical composition as defined in Claim 2, **characterized in that** the mixture of citric acid and disodium phosphate has a pH essentially equal to 3.

4. Pharmaceutical composition as defined in any one of Claims 1 to 3, **characterized in that** it contains Trimegestone and an oestrogen.

5. Pharmaceutical composition as defined in Claim 4, **characterized in that** the oestrogen is 17-beta-oestradiol, in particular in hemihydrate form.

6. Pharmaceutical composition as defined in Claim 4, **characterized in that** the oestrogen is Premarin.

7. Pharmaceutical composition as defined in any one of Claims 1 to 6, **characterized in that** it also comprises one or more binders.

8. Pharmaceutical composition as defined in Claim 7, **characterized in that** the binder is a cellulose derivative.

9. Pharmaceutical composition as defined in Claim 8, **characterized in that** the cellulose derivative is HPMC.

10. Pharmaceutical composition as defined in any one of Claims 1 to 9, **characterized in that** it also comprises one or more lubricants.

11. Pharmaceutical composition as defined in Claim 10, **characterized in that** the lubricant is stearic acid and/or talc.

12. Pharmaceutical composition as defined in any one of Claims 1 to 11, **characterized in that** it also comprises one or more opacifiers, in particular TiO₂.

13. Pharmaceutical composition as defined in any one of Claims 1 to 12, **characterized in that** it also comprises one of more chelating agents.

14. Pharmaceutical composition as defined in Claim 13, **characterized in that** the chelating agent is EDTA.

15. Pharmaceutical composition as defined in any one of Claims 1 to 14, **characterized in that** it also comprises one or more diluents, such as corn starch and/or lactose.

16. Pharmaceutical composition as defined in any one of Claims 1 to 15, **characterized in that** it comprises:
a) Trimegestone, optionally combined with an oestrogen, in particular oestradiol,
b) a buffer consisting of citric acid and disodium phosphate, the pH of which is essentially equal to 3,
c) where appropriate, one or more binders,
d) where appropriate, one or more lubricants,
e) where appropriate, one or more opacifiers,
f) where appropriate, one or more chelating agents,
g) one or more diluants.

17. Pharmaceutical composition as defined in any one of Claims 1 to 16, **characterized in that** it comprises (% by weight)
• 0.05% to 3% of Trimegestone
• 0.30% to 6% of oestrogen
• 0% to 6% of a binder, such as HPMC
• 0% to 2% of titanium dioxide
• 0.1% to 2% of a buffer consisting of citric acid and disodium phosphate, the pH of which is essentially equal to 3
• 0% to 4% of stearic acid and/or of talc
• 0% to 0.2% of a chelating agent, such as EDTA
• qs of diluant

18. Pharmaceutical composition as defined in Claim 17, **characterized in that** it contains from 0.2 to 0.8% of Trimegestone.

19. Pharmaceutical composition as defined in any one of Claims 1 to 18, **characterized in that** it comprises (% by weight)
• 0.4% of Trimegestone
• 3.2% of oestradiol
• 4% of HPMC
• 1% of titanium dioxide
• 0.15% of anhydrous disodium phosphate
• 0.4% of citric acid monohydrate
• 31% of corn starch
• 0.6% of stearic acid
• 1.2% of talc
• 0.1% of EDTA
• qs lactose

20. Pharmaceutical composition as defined in any one of Claims 1 to 18, **characterized in that** it comprises (% by weight)
• 0.8% of Trimegestone
• 3.2% of oestradiol
• 4% of HPMC
• 1% of titanium dioxide
• 0.15% of anhydrous disodium phosphate
• 0.4% of citric acid monohydrate
• 31% of corn starch
• 0.6% of stearic acid
• 1.2% of talc
• 0.1% of EDTA
• qs lactose

21. 65 mg pharmaceutical composition as defined in any one of Claims 1 to 18, **characterized in that** it comprises
• 0.25mg of Trimegestone
• 2.0 mg of oestradiol
• 2.5 mg of HPMC
• 0.6 mg of titanium dioxide
• 0.1 mg of anhydrous disodium phosphate
• 0.25 mg of citric acid monohydrate
• 20 mg of corn starch
• 38.1 mg of lactose
• 0.4 mg of stearic acid
• 0.8 mg of talc
• 0.065 mg of EDTA

22. 65 mg pharmaceutical composition as defined in any one of Claims 1 to 18, **characterized in that** it comprises
• 0.5 mg of Trimegestone
• 2.0 mg of oestradiol
• 2.5 mg of HPMC
• 0.6 mg of titanium dioxide
• 0.1 mg of anhydrous disodium phosphate
• 0.25 mg of citric acid monohydrate
• 20 mg of corn starch
• 37.85 mg of lactose
• 0.4 mg of stearic acid
• 0.8 mg of talc
• 0.065 mg of EDTA

23. Method of preparing the pharmaceutical compositions as defined in any one of Claims 1 to 22, **characterized in that** the procedure is carried out by wet granulation.

24. Method according to Claim 23, comprising the following steps:
1) obtaining a granule of oestrogen and Trimegestone
a) mixing the oestrogen and the Trimegestone with a binder and one or more diluants, then
b) wetting by adding an aqueous suspension containing the buffer as defined in any one of Claims 1 to 3, a binder and an opacifier
c) drying
d) calibrating
2) lubricating, and
3) compressing.

25. Method according to Claim 24, **characterized in that** two granules containing, respectively, Trimegestone and an oestrogen are prepared beforehand, the two granules being mixed prior to the "lubricating" step.

26. Method according to Claim 25, comprising the following steps:
1) obtaining a granule of oestrogen
a) mixing the oestrogen with a binder and one or more diluants, then
b) wetting by adding an aqueous suspension containing the buffer as defined in any one of Claims 1 to 3, a binder and an opacifier
c) drying
d) calibrating
2) obtaining a granule of Trimegestone
a) mixing a binder and one or more diluants, then
b) wetting by adding
- an aqueous suspension containing the buffer, a binder and an opacifier,
- the Trimegestone
c) drying
d) calibrating
3) mixing the granules of oestrogen and of Trimegestone
4) lubricating, and
5) compressing.

27. Blister pack comprising the tablets as defined in any one of Claims 1 to 22.

28. Blister pack as defined in Claim 27, **characterized in that** it is inserted into a bag under inert atmosphere, and in particular under nitrogen.

29. Use of Trimegestone optionally combined with an oestrogen, for preparing a pharmaceutical composition as defined in any one of Claims 1 to 22, intended for the treatment of symptoms of menopause and the prevention of osteoporosis.

30. Use, according to Claim 29, of Trimegestone optionally combined with an oestrogen, for preparing a pharmaceutical composition as defined in any one of Claims 1 to 22, intended for the treatment of symptoms of menopause in which said treatment is carried out by oral administration, according to any one of methods a) to e) :
a)
- of an oestradiol tablet, continuously, at a dose of 0.5 to 2 mg per day on the first 14 to 18 days,
- and of the tablet as defined in Claim 1 containing Trimegestone (0.025 to 2 mg per day) and oestradiol (0.5 to 2 mg per day) on the last 10 to 14 days of each 28-day cycle (cycles of 28 days without interruption between the cycles)
b)
- of an oestradiol tablet at a dose of 0.5 to 2 mg per day on the first 14 to 18 days,
- and of the tablet as defined in Claim 1 containing Trimegestone (0.025 to 2 mg per day) and oestradiol (0.5 to 2 mg per day) on the last 10 to 14 days, the treatment being stopped for 2 / 3 days per month at the end of each 28-day cycle (cycle of 28 days per month)
c)
- of the tablet as defined in Claim 1 containing Trimegestone (0.025 to 2 mg per day) and oestradiol (0.5 to 2 mg per day) on the first 10 to 14 days,
- and of an oestradiol tablet at a dose of 0.5 to 2 mg per day for the last 14 to 18 days, the treatment being administered either without interruption between each 28-day cycle, or with an interruption of 2 to 3 days per month at the end of each cycle
d)
- of an oestradiol tablet on the first 14 days, at a dose of 0.5 to 2 mg per day,
- and of the tablet as defined in Claim 1 containing Trimegestone (0.025 to 2 mg per day) and oestradiol (0.5 to 2 mg per day) during the last 11 days, the treatment being stopped for 5 to 6 days at the end of each 25-day cycle
e)
- of the tablet as defined in Claim 1 containing Trimegestone (0.025 to 2 mg per day) and oestradiol (0.5 to 2 mg per day), per day, without interruption of treatment.

31. Use of Trimegestone combined with an oestrogen, for preparing a pharmaceutical compososition as defined in any one of Claims 1 to 22, as a contraceptive.
